# EUROPEAN PATENT APPLICATION

(11) **EP 2 929 907 A1**
(43) Date of publication of application: **14.10.2015**
(21) Application number: 13876516.9
(22) Date of filing: 11.04.2013
(51) Int. Cl.: A61M 16/10

(54) **PORTABLE AIR PURIFIER**

(30) Priority: 01.03.2013 CN 201310065108
(71) Applicant: Hainan Wei-Kang Pharmaceutical (Qianshan) Company Limited, Anhui 246300 (CN)
(72) Inventor: WANG, Liuyi, Hefei City Anhui 230000 (CN); WANG, Jincan, Anqing City Anhui 246300 (CN); LIU, Haiyan, Anqing city Anhui 246300 (CN)
(74) Representative: Szabo, Zsolt
(86) International application number: PCT/CN2013/074061
(87) International publication number: WO 2014/131228

(57) **Abstract**

A portable air cleaner includes a host connected to a mask through a flexible pipe. The host includes a pressurizing and filtering chamber, an elementary air-storing chamber, a secondary air-storing chamber, a jet port and a valve. The pressurizing and filtering chamber includes a pump and a filtering unit placed therein. The elementary air-storing chamber includes a negative ion producer placed therein. The secondary air-storing chamber includes a pressure sensor, a temperature sensor and a temperature-changing element placed therein. The pressurizing and filtering chamber is connected to the elementary air-storing chamber through the jet port. The elementary air-storing chamber is connected to the secondary air-storing chamber through the valve.

## Description

### BACKGROUND OF THE INVENTION

The present invention relates to air cleaners and, more particularly, to a portable air cleaner.

An artificial respiration apparatus, such as CPAP and BIPAP, is used in a hospital or home. An artificial respiration apparatus without a central gas supply includes a filtering unit and a high-speed air supply placed in a shell. The highs-speed air supply is connected to a flexible pipe and a mask. The high-speed air supply includes a blower or a turbo charger. Air is sucked into the artificial respiration apparatus from the environment by vacuum. A user inhales the air from the artificial respiration apparatus through the flexible pipe. Thus, the user's respiration is facilitated by the artificial respiration apparatus. It is however difficult to control the air pressure produced by the artificial aspiration apparatus.

The filtering unit is used to filter out suspended particles from the air from the environment. After some time of use, the filtering unit is likely to be damaged and cannot be used to filter out suspended particles from the air. Thus, the artificial aspiration apparatus cannot be used to clean the air. There is nothing to instruct the user to clean the filtering unit or replace it with a new one. Hence, the user is likely to breathe in polluted air via the artificial aspiration apparatus. The artificial aspiration apparatus is harmful, instead of beneficial, to the user's health.

Moreover, the artificial aspiration apparatus cannot be used to improve the quality of air in any other way than filtering out suspended particles from the air. For example, the artificial aspiration apparatus cannot be used to heat or cool the air.

### BRIEF SUMMARY OF THE INVENTION

It is the primary objective of the present invention to obviate the problems encountered in the prior art by providing a portable air cleaner to deal with a deteriorating problem of pollution related to PM2.5 particles to reduce the risk of inhaling of harmful substances into human bodies in working environments.

To achieve the foregoing objective, the portable air cleaner includes a host connected to a mask through a flexible pipe. The host includes a pressurizing and filtering chamber, an elementary air-storing chamber, a secondary air-storing chamber, a jet port and a valve. The pressurizing and filtering chamber includes a pump and a filtering unit placed therein. The elementary air-storing chamber includes a negative ion producer placed therein. The secondary air-storing chamber includes a pressure sensor, a temperature sensor and a temperature-changing element placed therein. The pressurizing and filtering chamber is connected to the elementary air-storing chamber through the jet port. The elementary air-storing chamber is connected to the secondary air-storing chamber through the valve. The pressure sensor is used to measure air pressure in the secondary air-storing chamber and control the valve based on the measurement.

In a further aspect, the pump is a medical jet pump that includes a jet pipe placed in a jet port. The medical jet pump consumes a little electricity to process a lot of air to satisfy the need of crowded pedestrians.

In a further aspect, the filtering unit includes an elementary filter, a middie-range filter and a high-end filter placed at an entrance of the pressurizing and filtering chamber. The filters can quickly be detached, washed and replaced. Regarding the screen size, the elementary filter is larger than the middle-range filter that is larger than the high-end filter. The screen size of the high-end filter is smaller than 2.5 micrometers so that it can filter out PM2.5 particles.

In a further aspect, the portable air cleaner includes a gauge and clock placed in the flexible pipe to measure the flow of air processed by the air cleaner and the working hours of the host. When the working hours and the flow reach predetermined upper limits, the host notifies a user of washing and replacing the filters.

In a further aspect, the portable air cleaner according includes a check valve placed in a connection of the flexible pipe to the mask. The mask is shaped in compliance with a nose to avoid blocking the user's nose and mouth all together that would otherwise interfere with the user's eating and speaking. The check ensures a one-way flow of air to prevent exhaust from entering the flexible pipe.

In a further aspect, the host includes a single-chip microcomputer and a rechargeable power supply for saving power and recharging.

In further aspect, the host includes a switch and an LED placed on a shell thereof to show the flow processed by the air cleaner, the pressure, the temperature and the amount of electricity in the rechargeable power supply.

In another aspect, the host includes a touch panel placed on a shell thereof to facilitate the operation of the air cleaner.

### DESCRIPTION OF THE DRAWINGS

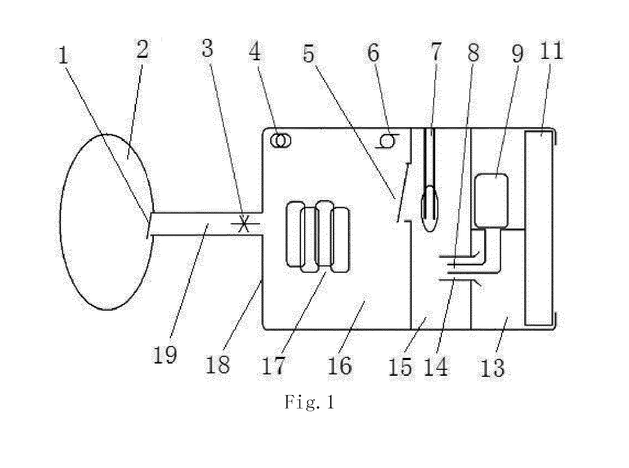

FIG. 1 is a block diagram of a portable air cleaner according to the preferred embodiment of the present invention.

### DETAILED DESCRIPTION OF THE INVENTION

To render the techniques, features, objectives and effects of the present invention easily understandable, the present invention will be described via detailed illustration of the preferred embodiment thereof referring to the drawing.

Referring to FIG. 1, a portable air cleaner includes a mask 2 connected to a host 18 through a flexible pipe 19. The host 18 includes a pressurizing and filtering chamber 13, an elementary air-storing chamber 15 and a secondary air-storing chamber 16. The pressurizing and filtering chamber 13 includes a filtering unit 11 and a pump 9 placed therein. The elementary air-storing chamber 15 includes a negative ion producer 7 placed therein. The secondary air-storing chamber 16 includes a temperature-changing element 17, a pressure sensor 4 and a temperature sensor 6 placed therein. The pressurizing and filtering chamber 13 is connected to the elementary air-storing chamber 15 through a jet port 14. The pump 9 is a medical jet pump that includes a jet pipe 8 placed in the jet port 14. The medical jet pump is efficient and consumes a little electricity to provide a lot of air to satisfy the need of crowded pedestrians efficient. The elementary air-storing chamber 15 is connected to the secondary air-storing chamber 16 via a valve 5. The pressure sensor 4 is used to measure the air pressure in the secondary air-storing chamber 16 and control the valve 5 based on the measurement.

A flexible pipe 19 includes a gauge and clock 3 placed therein. The filtering unit 11 is in the vicinity of an entrance (not numbered) of the pressurizing and filtering chamber 13. The gauge and clock 3 is used to detect the flow of air processed by the host 18 and the time taken by the host 18. Thus, the flow of the air that is processed by the host 18 can be determined and shown on a display (not shown). When the flow reaches a predetermined upper limit, the host 18 provides an alert to instruct a user to clean the filtering unit 11 or replace the filtering unit 11 with a new one. A check valve 1 is placed in a connection of the flexible pipe 19 with the mask 2. The mask 2 is shaped in compliance with the human nose. The mask 2 is used to cover the user's nose, not the mouth. Thus, the user can breathe while eating or speaking. The check valve 1 is used to ensure that air travels into the mask 2 from the flexible pipe 19, not vice versa. That is, exhaust from the user cannot travel into the flexible 19 from the mask 2. A touch panel is placed on a shell of the host 18. The flow, the time, the temperature and the amount of electricity in a battery used in the host 18 can be shown on the touch panel.

The present invention has been described via the detailed illustration of the preferred embodiment. Those skilled in the art can derive variations from the preferred embodiment without departing from the scope of the present invention. Therefore, the preferred embodiment shall not limit the scope of the present invention defined in the claims.

## Claims

1. A portable air cleaner including a host, a mask and a flexible pipe for connecting the host to the mask, wherein the host includes a pressurizing and filtering chamber including a pump and a filtering unit placed therein, an elementary air-storing chamber including a negative ion producer placed therein, a secondary air-storing chamber including a temperature-changing element, a pressure sensor and a temperature sensor placed therein, wherein the filtering chamber is connected to the elementary air-storing chamber via a jet port, wherein the elementary air-storing chamber is connected to the secondary air-storing chamber via a valve.

2. The portable air cleaner according to claim 1, wherein the pump is a medical jet pump, wherein the pressurizing and filtering chamber includes a jet pipe including an end connected to the pump and another end inserted in the jet port.

3. The portable air cleaner according to claim 1, wherein the filtering unit includes an elementary filter, a middle-range filter and a high-end filter placed at an entrance of the pressurizing and filtering chamber.

4. The portable air cleaner according to claim 3, wherein the flexible pipe includes a gauge and clock placed therein.

5. The portable air cleaner according to claim 3, including a check valve 1 placed in a connection of the flexible pipe to the mask.

6. The portable air cleaner according to claims 1-5, wherein the host includes a single-chip microcomputer and a rechargeable power supply.

7. The portable air cleaner according to claim 6, wherein the host includes a switch and an LED placed on a shell thereof.

8. The portable air cleaner according to claim 6, wherein the host includes a touch panel placed on a shell thereof.
